# EUROPEAN PATENT APPLICATION

(11) **EP 2 062 906 A1**
(43) Date of publication of application: **27.05.2009**
(21) Application number: 07121317.7
(22) Date of filing: 22.11.2007
(51) Int. Cl.: C07F 15/00, B01J 31/12, C07B 53/00

(54) **Coordination complex system comprising tautomeric ligands**

(71) Applicant: Universiteit van Amsterdam, 1000 GG Amsterdam (NL)
(72) Inventor: Reek, Joost Nikolaas Hendrik, 3817 BK Amersfoort (NL); Patureau, Frederic, 78 112 Fourqueux (FR); Kuil, Mark, 3931 TT Leusden (NL); Sandee, Albertus Jacobus, 3522 EN Utrecht (NL)
(74) Representative: Metman, Karel Johannes

(57) **Abstract**

The invention relates to a coordination complex system comprising a ligand (1) having the formula: R₁-SO₂-NH-P(XR₂)₂ (1a) ; or R₁-SO₂-N=PH(XR₂)₂ (1b); or R₁₋SO(OH)=N-P(XR₂)₂ (1c) ; wherein X is independently O, S, NH, or a bond; R₁ and R₂ are independently selected from substituted or unsubstituted alkyl and aryl; wherein at least one equivalent of a ligand (1) is complexed to a metal selected from a transition metal and lanthanide; wherein Y is O, S, NH, or a bond.
The invention also relates to the use of said coordination complexes as catalysts in the hydroformylation, hydrogenation, transfer hydrogenation, hydrocyanation, polymerization, isomerization, carbonylation, cross-coupling, metathesis, CH activation, allylic substitution, aldol condensation, or Michael addition.

## Description

The invention relates to a coordination complex system comprising a ligand which is complexed to at least a metal selected from a transition metal or lanthanide, to a catalyst system comprising said coordination complex system, and to the use of said coordination complex system as a catalyst.

Bidentate ligands represent an important class of ligands for transition metal catalysis, however, their synthesis is often tedious and time consuming compared to their monodentate counterparts. Combinatorial approaches to catalyst discovery are therefore mostly based on monodentate ligands. In WO 2004/103559 supramolecular ligands have been identified as a new class of ligands that form by a self-assembly process of ligand building blocks through specific interactions. These ligands have bidentate character, are modular in nature and are synthetically accessible, and therefore highly promising when applied in combinatorial approaches for rapid catalyst discovery. Here we disclose, a new generation of hydrogen bonded supramolecular multidentate ligands based on sulfonamide derivatives that are adaptive to the environment of transition metals, by means of tautomerization. The invention pertains to a coordination complex system comprising a ligand (1) having the formula: R₁-SO₂-NH-P(XR₂)₂ (1a); or R₁-SO₂-N=PH(XR₂)₂ (1b); or R₁-SO(OH)=N-P(XR₂)₂ (1c); wherein X is independently O, S, NH, or a bond; R₁ and R₂ are independently selected from substituted or unsubstituted alkyl and aryl; wherein at least one equivalent of a ligand (1) is complexed to a metal selected from a transition metal and lanthanide; wherein Y is O, S, NH, or a bond.

The terms "ligand (1)" or simply "(1)" will further mean the compound that may have formula (1a), (1b), (1c), or a mixture thereof.

Preferred R₁ and R₂ are C1-C6 alkyl, phenyl, naphthyl and the like, which are unsubstituted or substituted with at least one of alkyl, aryl, nitro, and halogen groups. R₂ and R₄ are preferably bisphenyl or bisnaphthyl, or an annelated bisaromatic system, including N-containing aromatic systems. Preferably the complex comprises at least two ligands, wherein X is preferably a bond in one of the ligands and preferably oxygen in one of the other ligands. It is also possible that in a certain ligand one of the moieties X is a bond and the other X in the same ligand is oxygen. It is also preferred that in a certain ligand one of the groups R₂ is an alkyl group whereas the other R₂ is an aryl group.

The ligands of the invention can be prepared by a simple condensation reaction of a sulfonamide, for instance para-n-butylphenylsulfonamide to a phosphine halide, such as Ph₂PCl. In solution these sulfonamide phosphorous compounds may exist in different tautomeric forms, i.e. (1a), (1b), and (1c). These tautomeric forms can in some cases be detected as the different tautomers are in slow exchange on the NMR time scale. The tautomerization is crucial for its coordination behavior and gives rise to the formation of hydrogen bonded homo- and hetero-bidentate ligands, and the corresponding metal complexes are for instance found to be very efficient in the asymmetric catalytic processes.

The three tautomers of the ligand (1) (1a, 1b, 1c) and formation of complex (3) with Rh(acac)(CO)₂ is shown in Scheme 1. In this description the term ligand (2) means a second ligand having the formula of ligand (1), which second ligand may the same or different as the first ligand.

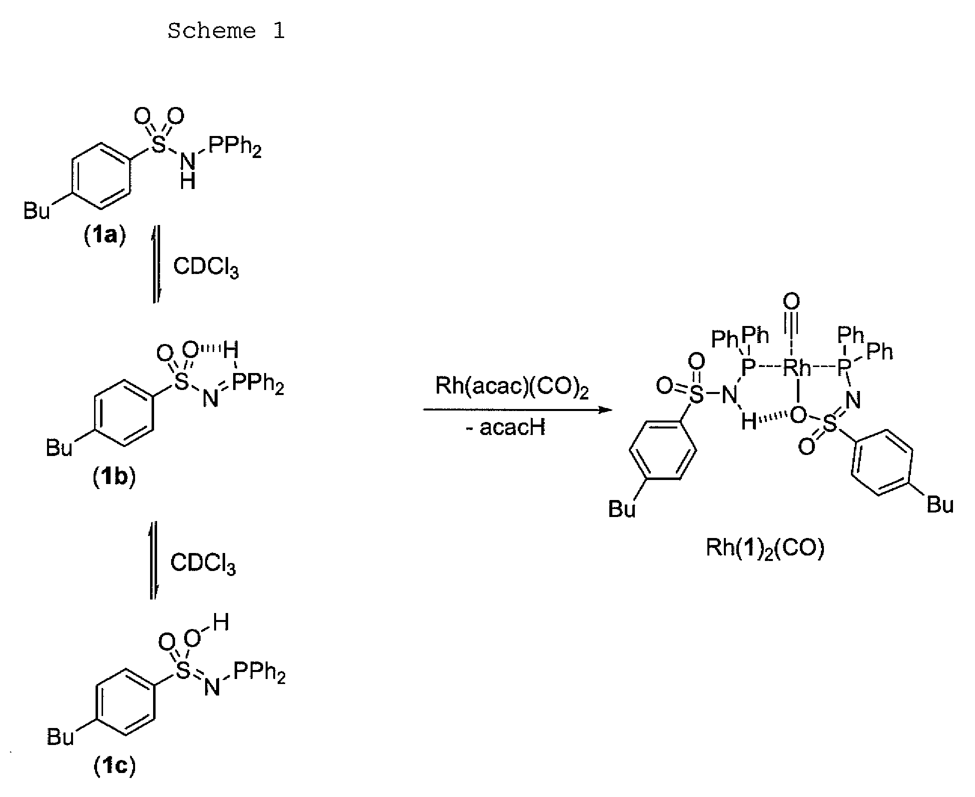

The addition of two equivalents of (1) to a CDCl₃ solution of Rh(acac)(CO)₂ gives a complex with two different ligands coordinated in trans position about the metal center as is shown in the typical AB pattern in ³¹P NMR with large P-P coupling. The deprotonated form of ligand (1c) likely coordinates to the metal in a P-O chelate fashion, which is also observed by dft-calculations. The ligand tautomers (1a) and (1c) at the metal center can be identified through their coupling pattern in NMR.

The hydrogen bond interaction between ligands (1a) and (1c) coordinated to the metal can be confirmed by IR spectroscopy showing the concentration independent NH vibration at 3281 cm⁻¹ which is typically shifted compared to the free NH (3341 cm⁻¹). Using rhodium as metal the NH NMR shift of metal(1a,1c)(CO) is concentration independent, whereas the free ligand (1a) shows concentration dependent NMR shift of the NH proton. This indicates that the free tautomer (1a) self-associates at higher concentrations.

Hetero-ligated metal complexes can be made by complexing two different ligands to the metal. For instance, a ligand (2) can be prepared from bisnaphthol-PCl and a sulfonamide giving only one tautomer in ³¹P NMR. As shown in Scheme 2 in a CDCl₃ NMR solution of (1), (2) and Rh(acac)(CO)₂ in a 1:1:1 ratio pure Rh(2.1)(CO) (complex 4) was obtained with the ligands in a trans geometry as was evidenced with the typical AB system in the ³¹P NMR.

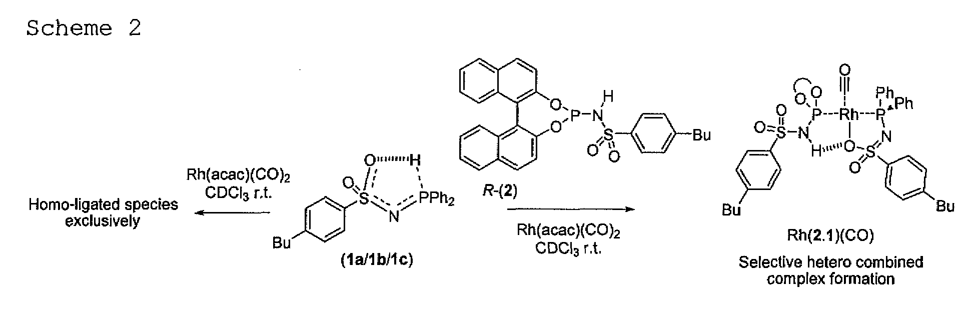

The various rhodium complexes based on homo- and hetero-ligands can be applied in the asymmetric hydrogenation reaction. All reactions went to completion after 8 h, also when only 0.1 mole% catalyst was applied (entries 8-14, Table 1). Whereas complexes based on ligand (2) provided the product in the highest ee (enantiomeric excess 99%, entry 10), the hetero-ligand complex 1. (2) was found to be the most active (TOF 1.2·10³ mole/mole/h) and also selective (92% ee). All other mixed ligand systems using PPh₃ or aniline-S-(-)-1,1'-bis-2-naphthol-phosphor-amidite (5), provided the product with much lower enantioselectivity. Kinetic experiments using Rh(2.1) complexes as the catalyst indicate that the reaction is zero order in molecular hydrogen (between 4-16 bar) and zero order in substrate (between 0.040 and 0.20 M) and is first order in rhodium (between 0.025 and 0.10 mM). The mechanism is believed to be as depicted in Scheme 3. A hetero-ligated Rhodium(I) complex (resting state) gives a rate limiting intermolecular oxidative addition to give Rh(III) and one anionic ligand.

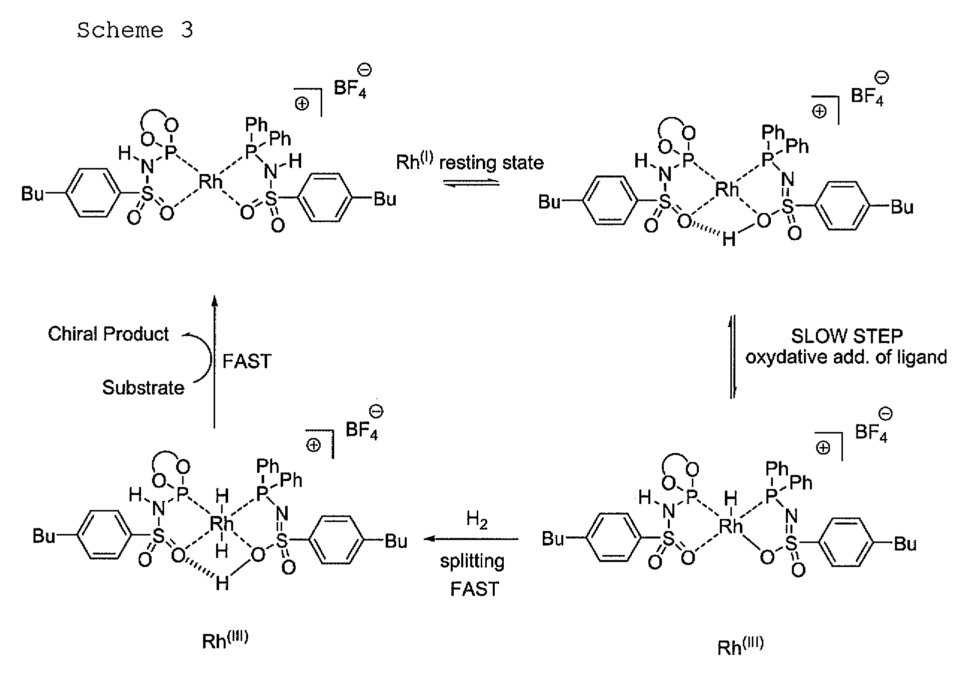

Subsequently, heterolytic cleavage of molecular hydrogen leads to the rhodium(III)dihydride that reacts in the known manner with the substrate to form the chiral product.

Complexes of the type [Metal(1)(2)]⁺(BF₄⁻)can be used in the rhodium catalyzed asymmetric hydrogenation of alkenes to provide enantiomerically enriched products. Complexes of the type Metal(1)(2)(CO) can be used in the Rh catalyzed hydroformylation of alkenes. Complexes of the type Metal(1)(2)(CO) can be used in polymerization reactions, such as the Rh catalyzed polymerization of carbenes (for an example of this reaction using other catalysts see: Hettersheid, D. G. H.; et al.; J. Am. Chem. Soc., (128), No. 30, 2006, 9746-9752. The Rh-catalyzed polymerization of EDA (Ethyl-Diazoacetate) yielded the corresponding polymer with high molecular weight (Mw up to 475 kDa).

The coordination of ligand (1) with other transition metals such as PdCl₂(CH₃CN)₂ was also demonstrated. The complexation of two equivalents of ligand (1) led to the formation of PdCl₂(1)₂. Upon addition of 8 equivalents of Et₃N, the ³¹P signal showed the expected AB system attributed to PdCl(1a.1c) with the ligands coordinated in a cis-fashion. Complexation behaviour to platinum is similar. This shows that the complexes of the invention can be prepared with different metals.

The coordination complex system can be used as a catalyst, for example for hydroformylation, hydrogenation, transfer hydrogenation, hydrocyanation, polymerization, isomerization, carbonylation, cross-coupling, metathesis, CH activation, allylic substitution, aldol condensation, or Michael addition.

The invention is further illustrated by the following non-limitative examples.

### General

Methyl-2-acetamidoacrylate, R-(+)-1,1'-bis-2-naphthhol, chlorodiphenylphosphine, phosphorus trichloride, 4-butylbenzene-1-sulfonamide, were purchased from Aldrich, Reuter Chemische Apparatbau, Aldrich, Aldrich, and Maybridge respectively. Rh(acac)(CO)₂ and Rh(nbd)₂BF₄ were purchased from Merck and Alfa respectively. Styrene, 1-octene, decane, and EDA (ethyl-diazoacetate) were all purchased from Aldrich. Synthesis of N-(1-Phenyl-vinyl)-acetamide and related substrates are according: van den Berg, M. et al; Adv. Synth. Catal., 2002, 344, 1003-1007.

All reactions were carried out in dry glassware under argon or nitrogen atmosphere. Every solution addition or transfer was performed via syringes. All solvents were dried and distilled with standard procedures.

Chromatographic purifications were performed by flash chromatography on silica gel 60-200 µm, 60 Å, purchased from Screening Devices. Nuclear Magnetic Resonance experiments were performed on a Varian Inova spectrometer (¹H_{:} 500 MHz, ³¹P: 202.3 MHz, ¹³C: 125.7 MHz). Chemical shifts are referenced to the solvent signal (7.27 ppm in ¹H and 77.0 ppm in ¹³C NMR for CDCl₃). Conversions and enantiomeric excess for the asymmetric hydrogenation of Methyl-2-acetamidoacrylate were determined by Gas Chromatography on a Phase PH MDEX - FT: 0.1um, L: 5 m, id: 0.1 mm. Conversions and enantiomeric excess for the asymmetric hydrogenation of dimethyl itaconate were determined by G.C. on a Supelco BETA DEX column (0.25 mm x 30 m). Conversion and yields of the hydroformyation products were determined on a Shimadzu GC-17A apparatus (split/splitless, equipped with F.I.D. detector and a BPX35 column (internal diameter of 0.22 mm, film thickness 0.25 µm, carrier gas 70 kPa He)). Molecular distributions were measured using size exclusion chromatography (SEC) on a Shimadzu LC-20AD system with Waters Styragel HR1, HR2 and HR4 columns in series and a Shimadzu RID-10A refractive index detector using CH₂Cl₂ as mobile phase at 1 mL/min and T=313 K. Calibration was done using polystyrene standards in the range of 760 to 1880000 g/mol purchased from Aldrich.

Aniline-S-(-)-1,1'-bis-2-naphthhol-phosphoramidite: Ligand (5) was prepared according to literature procedure (Lefort, L.; et al., Org. Lett., 2004, 6, 1733-1735).

Hydrogenation substrate: N-(1-phenyl-vinyl)-acetamide was prepared according to literature procedures.

### Example 1

### 4-Butylbenzene-1-sulfonamide-diphenylphosphine

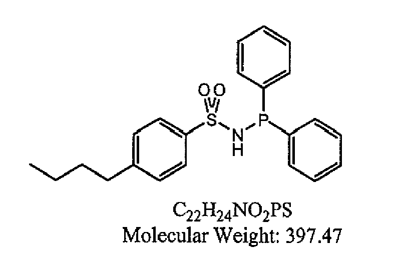

Commercially available 4-butylbenzene-1-sulfonamide (9.376 mole, 1 eq) was placed in a Schlenk flask under Argon. 10 mL of toluene were dropped on it leading to a blurred suspension which was stirred at room temperature for 5 minutes. The solvent was then evaporated. The process was repeated twice. The compound was then dissolved in tetrahydrofuran (20 mL) and triethylamine (25 mmole), leading to a clear colorless solution. Distilled chlorodiphenylphosphine (9.376 mmole, 1.0 eq) was added dropwise under strong magnetic stirring at room temperature. The immediate formation of salt was observed as a white suspension. The suspension was left to stir overnight at room temperature. The suspension was then filtered under argon atmosphere and the resulting clear solution was evaporated to a white solid. The product was redissolved in 10 mL of diethylether leading to a clear solution. 10 mL of pentane were added dropwise to the solution causing a white solid to crash out. The organic layer was syringed out, and the white solid obtained was washed twice with 10 mL of pentane. The product was filtered over a short silica gel column with dichloromethane as eluent. The fractions containing the product where combined and evaporated yielding the ligand (1) as a white solid with a quantitative yield.

### Example 2

### 4-Butylbenzene-1-sulfonamide-R-(+)-1,1'-bis-2-naphthol-phosphoramidite

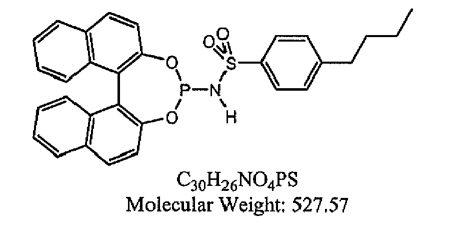

Commercially available R-(+)-1,1'-bis-2-naphthol (9.647 mmole, 1.03 eq) was placed in a Schlenk flask under argon. 10 mL of toluene were added to it leading to a suspension which was stirred for 10 minutes at room temperature. The solvent was then evaporated, and the process repeated twice. 36 mL of toluene were finally added to the white solid along with trichlorophosphine (12 mL, distilled), leading to a suspension. The mixture was gently heated up to 80 °C yielding a clear colorless solution which was left to stir overnight. The solution was then cooled down to room temperature and evaporated leading to white sticky foam. The product was redissolved in toluene (20 mL) and evaporated again. The sticky foam obtained was then dissolved in 10 mL of tetrahydrofuran leading to a clear colorless solution.

In a second Schlenk flask was placed commercially available 4-butylbenzene-1-sulfonamide (9.376 mmole, 1 eq) under argon atmosphere. The amide was then submitted to 10 mL of toluene leading to a suspension stirred at room temperature for 10 minutes. The solvent was evaporated and the process was repeated twice. The amide was then dissolved in tetrahydrofuran (25 mL) and triethylamine (57 mmole) leading to a clear colorless solution. The solution of R-(+)-1,1'-bis-2-naphthol-PCl previously prepared was added drop wise to the amide solution under strong magnetic stirring at room temperature. The suspension obtained was left to stir overnight at room temperature. The suspension was then filtered under argon atmosphere, and the clear colorless solution collected was evaporated leading to white foam. The product was redissolved in dichloromethane (10 mL), and re-evaporated to a white foam. 10 mL of toluene were then added leading to a thick solution. Within a few seconds however, a white solid broke out under the form of what appeared to be small white crystals. An extra 50 mL of toluene were added leading to a suspension which was filtered under argon atmosphere. The white product obtained was recovered from the filter by redissolving in dichloromethane and the clear solution obtained was evaporated. The ligand (2) was obtained with a quantitative yield as a thin white powder.

### Rh(1a.1c)(CO): complex 3:

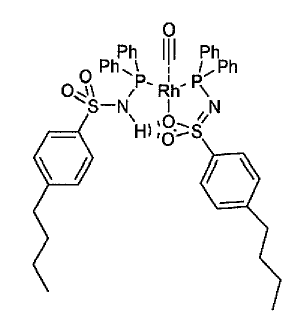

Typically: Ligand (1) (0.03501 mmole, 2.00 eq) and commercially available Rh(acac)(CO)₂ (0.01745 mmole, 1.00 eq) were placed in a small Schlenk tube under argon atmosphere. CDCl₃ (4.1404 g, 2.7603 mL) was dropped on them leading to a clear yellow solution (6.325 mM of [Rh]). 600 µL of this solution were transferred to the NMR tube under argon conditions.

In a similar fashion, complex Rh(1)₂(CO)(complex 3) was studied in toluene-D8. All the characteristic couplings were found identical, showing that the solvent has little influence on the conformation and angles of the H-bonded supramolecular multidentate structure.

The identification of the NH signal of the Rh(1)₂(CO) structure was proven with a H-P 2D correlation spectra set at J_{H-P} = 16 Hz. A solution of Rh(1)₂(CO) solution (9.959 mM in CDCl₃) was prepared as previously described and the H-P correlation was measured (¹H NMR 500 MHz, 223K).

### Rh(2.1c)(CO): complex 4:

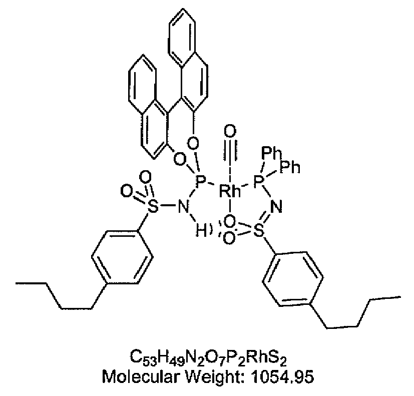

Ligand (1) (0.01957 mmole, 0.99 eq), ligand (2) (0.02036 mmole, 1.03 eq), and Rh(acac)(CO)₂ (0.01970 mmole, 1.00 eq) were placed in a small Schlenk tube under argon atmosphere. CDCl₃ (540 µL) was added leading to a clear bright yellow solution, which was transferred in an NMR tube under argon atmosphere. A typical AB system was obtained in ³¹P NMR: complex (4), with 95% purity.

### Example 3

Comparative experiment with aniline-S-(-)-1,1'-bis-2-naphthol-phosphoramidite: as the H-bond donor ligand 5:

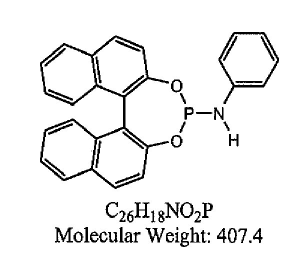

Ligand (1) (0.00704 mmole, 1.00 eq), ligand 5 (0.00703 mmole, 1.00 eq), and Rh(acac)(CO)₂ (0.00704 mmole, 1.00 eq) were placed in a small Schlenk tube under argon atmosphere. CDCl₃ (1.1097g, 740 µL) was dropped on it leading to a clear orange solution. The solution was transferred to the NMR tube under argon atmosphere. The ³¹P NMR profile afforded only homo-combined coordinated species.

### General procedure for the asymmetric hydrogenation of methyl-2-acetoamidoacrylate, dimethyl itaconate and N-(1-phenyl-vinyl)-acetamide:

Typically: a 15 mL pressure reactor containing a glass insert was subsequently charged, under inert conditions, with 0.50 mL of a solution of ligand (1), 0.50 mL of a solution of ligand (2), 0.50 mL of solution of Rh(nbd)₂BF₄, and 1.0 mL of solution of substrate, all of dichloromethane at the required concentrations. The 2.5 mL resulting solution was then stirred at room temperature for 5 minutes. The solution was then exposed to 10 bars of H₂ atmosphere, and left to stir at room temperature (298 K) for 8 hours (vortex-type stirring: 400 rpm). Conversions and enantio-selectivities were determined by chiral GC.

### General procedure for the asymmetric hydrogenation of methyl-2-acetoamidoacrylate with monitoring gas uptakes:

The experiments were carried out in the AMTEC SPR16 (www.amtec-chemnitz.de) consisting of 16 parallel reactors equipped with temperature and pressure sensors, and a mass flow controller. The apparatus is suited for monitoring gas uptake profiles during the catalytic reactions.

Three autoclaves were heated to 90 °C and flushed with argon (22 bar) five times. Next the reactors were cooled to 25 °C and flushed again with argon (22 bar) five times. The autoclaves were charged with 0.80 µmol of [Rh(nbd)₂(BF₄)] and 1.92 µmol of phosphorus ligand(s) in 3.00 ml of dichloromethane under argon. The reaction mixtures were mixed for 30 minutes at 25 °C, before 800 µmol of the substrate in 5.0 ml of dichloromethane were added under argon. The reactors were pressurized with 10 bar H₂ and the pressure was kept constant during the whole reaction. The reaction mixtures were stirred at 25 °C for 13 h and the hydrogen uptake was monitored and recorded for every reactor. After catalysis the pressure was reduced to 2.0 bar and samples (0.3 ml) were taken.

In Table 1 an overview is given for some Rh-catalyzed asymmetric hydrogenation of methyl-2-acetamido-acrylate.

**Table 1: hydrogenation of methyl-2-acetoamidoacrylate, entry 4 and 11 have been added as control experiments.**

| **entry** | **combined ligands** | **[Rh] (mM)** | **Substrate/Rh** | **ee %** | **R / S** | **T.O.F.** |
|---|---|---|---|---|---|---|
| 1 | (1) / (1) | 1 | 100 | 0.0 | - | - |
| 2 | (1) / (2) | 1 | 100 | 90.6 | S | - |
| 3 | (2) / (2) | 1 | 100 | 95.8 | S | - |
| 4 | PPh₃ / PPh₃ | 1 | 100 | 0.0 | - | - |
| 5 | PPh₃ / (5) | 1 | 100 | 13.3 | S | - |
| 6 | (5) / (5) | 1 | 100 | 62.1 | R | - |
| 7 | (5) / (1) | 1 | 100 | 41.7 | R | - |
| 8 | (1) / (1) | 0.1 | 1000 | 0.0 | - | 6.5·10² |
| 9 | (1) / (2) | 0.1 | 1000 | 91.7 | S | 1.2·10³ |
| 10 | (2)/(2) | 0.1 | 1000 | 99.0 | S | 5.7·10² |
| 11 | PPh₃ / PPh₃ | 0.1 | 1000 | 0.0 | - | - |
| 13 | PPh₃ / (5) | 0.1 | 1000 | 3.6 | S | - |
| 13 | (5) / (5) | 0.1 | 1000 | 65.0 | R | - |
| 14 | (5) / (1) | 0.1 | 1000 | 46.0 | R | - |

| | | | | | | |
|---|---|---|---|---|---|---|
| Rh/ (L1+L2) = 1/ 2.4 in CH₂Cl₂, 10 bars H₂, 8h at 298 K, full conversion for all entries, ee. determined by chiral GC, T.O.F. ((mol/mol)/h) determined from gas uptake profiles at 20 % conversion. | | | | | | |

**Table 2: hydrogenation of Dimethyl Itaconate.**

| **entry** | **combined ligands** | **[Rh] (mM)** | **conversion %** | **ee %** | **R / S** |
|---|---|---|---|---|---|
| 1 | (1) / (1) | 1 | 100 | 0.0 | - |
| 2 | (1) / (2) | 1 | 96.5 | 51.6 | R |
| 3 | (2) / (2) | 1 | 15.2 | 48.3 | R |

| | | | | | |
|---|---|---|---|---|---|
| Rh/ (L1+L2) = 1/ 2.6 in CH₂Cl₂, 10 bars H₂, 8h at 298 K, Substrate/Rh = 100, ee. determined by chiral GC. | | | | | |

**Table 3: hydrogenation of N-(1-Phenyl-vinyl)-acetamide.**

| **entry** | **combined ligands** | **[Rh] (mM)** | **conversion %** | **ee %** | **R/S** |
|---|---|---|---|---|---|
| 1 | (1) / (2) | 1 | 100.0 | 56.0 | R |
| 2 | (2) / (2) | 1 | 77.0 | 48.7 | R |

| | | | | | |
|---|---|---|---|---|---|
| Rh/ (L1+L2) = 1/ 2.4 in CH₂Cl₂, 10 bars H₂, 16h at 298 K, Substrate/Rh = 100, ee. determined by chiral GC. | | | | | |

### General procedure for the Rh-catalyzed hydroformylation of styrene:

Rh(acac)(CO)₂ (0.0031 mmole, 1 eq), Ligand (1)(0.0239 mmole,7.7 eq), toluene (2.5 mL), Styrene(previously filtered over basic alumina, 3.002 mmole, 968 eq) and decane (internal standard, 0.990 mmole) were placed in a small Schlenk tube under argon, leading to a clear yellow solution. The resulting solution was placed in a suited 5mL glass reactor equipped with a matching stirring bean, which was placed inside the autoclave. The autoclave was purged thrice with 15 bars of H₂/CO (1:1), then submitted to 15 bars of H₂/CO for 20.5 h at 333K. The autoclave was then cooled down in an ice-bath, depressurized and a sample was prepared for the GC. A conversion of 22.9 % was obtained, with 10.27 b/l (branched/linear aldehyde product).

### General procedure for the Rh-catalyzed hydroformylation of 1-octene:

Rh(acac)(CO)₂ (0.0013 mmole, 1 eq), Ligand (1) (0.0123 mmole, 9.5 eq), toluene (1.95 mL) and decane (internal standard, 4.2019 mmole) were place inside a 15 mL reactor autoclave with a suited magnetic bean under argon atmosphere. The resulting solution was incubated for 56 min at 343K and 10 bars of H₂/CO (1:1). The 1-octene (previously filtered over basic alumina, 8.3577 mmole, 6429 eq) was added in a toluene solution (2.14 mL) via syringe. The resulting solution was submitted to 10 bars of H₂/CO at 343 K for 4 hours under strong magnetic stirring. The autoclave was then cooled with an ice bath, depressurized and a sample was injected on GC. The conversion was found at 10.74% (isomers : 28.23%, linear aldehyde: 49.62%) and 2.24 as linear/branched aldehyde ratio.

### General procedure for the Rh-catalyzed polymerization of EDA (Ethyl-Diazoacetate):

Rh(acac)(CO)₂ (0.0774 mmole, 1 eq), Ligand (1) (0.1628 mmole, 2.1 eq) were placed under argon in a small Schlenk tube. CH₂Cl₂ (10 ml) were added leading to a clear yellow solution. EDA (4.34 mmole, 56 eq) was then added under strong magnetic stirring at room temperature. Immediately a strong degassing was observed, and the resulting orange solution was left to stir overnight at room temperature. The solvent was then evaporated to a sticky product. Cold MeOH (10 mL) was then added leading to a white suspension into an orange solution. The product was centrifuged, and the MeOH removed. The polymer was washed twice more with cold MeOH, and then submitted to vacuum. The white solid obtained (isolated yield : 3.5%) was analyzed on GPC and yielded a Mw/Mn of 18.5 for a Mw of 475 kDa.

## Claims

1. A coordination complex system comprising a ligand (1) having the formula:
R₁-SO₂-NH-P(XR₂)₂ (1a); or R₁-SO₂-N=PH(XR₂)₂ (1b); or R₁-SO(OH)=N-P(XR₂)₂ (1c); wherein X is independently O, S, NH, or a bond; R₁ and R₂ are independently selected from substituted or unsubstituted alkyl and aryl; wherein at least one equivalent of a ligand (1) is complexed to a metal selected from a transition metal and lanthanide; wherein Y is O, S, NH, or a bond.

2. The coordination complex system of claim 1 wherein R₁ and R₂ are independently selected from C1-C6 alkyl, phenyl, and naphthyl, which are unsubstituted or substituted with at least one of alkyl, aryl, nitro, and halogen.

3. The coordination complex system of claim 1 or 2 wherein R₁ is phenyl, which is unsubstituted or substituted with at least one of alkyl, aryl, nitro, and halogen.

4. The coordination complex system of any one of claims 1 to 3 wherein R₂ is independently selected from bisphenyl and bisnaphthyl, which groups may optionally have one or more N atoms in the aromatic ring.

5. The coordination complex system of any one of claims 1 to 4 wherein at least two equivalents of ligand (1) are complexed to a metal, at least one ligand having X is a bond and at least one ligand having X is O.

6. The coordination complex system of any one of claims 1-5 wherein the transition metal is rhodium or palladium.

7. The coordination complex system of any one of claims 1-6 wherein at least one of the ligands is immobilized onto an inorganic support, a polymeric organic support, or a hybrid support.

8. A catalyst system comprising the coordination complex system of any one of claims 1-7.

9. Use of the coordination complex system of any one of claims 1-7 as a catalyst.

10. The use according to claim 9 for hydroformylation, hydrogenation, transfer hydrogenation, hydrocyanation, polymerization, isomerization, carbonylation, cross-coupling, metathesis, CH activation, allylic substitution, aldol condensation, or Michael addition.
